# FASCICULE DE BREVET EUROPEEN

(11) **EP 4 104 890 B1**
(45) Date de publication et mention de la délivrance du brevet: **25.02.2026**
(21) Numéro de dépôt: 22179472.0
(22) Date de dépôt: 16.06.2022
(51) Int. Cl.: A61M 39/02

(54) **CHAMBRE IMPLANTABLE ET NÉCESSAIRE ASSOCIÉ**
IMPLANTIERBARE KAMMER UND ENTSPRECHENDES NECESSAIRE
IMPLANTABLE CHAMBER AND ASSOCIATED KIT

(30) Priorité: 17.06.2021 FR 2106450
(43) Date de publication de la demande: 21.12.2022
(73) Titulaire: Vygon, 95440 Ecouen (FR)
(72) Inventeur: WALTER, Thomas, 92500 Rueil Malmaison (FR); CARPENTIER, Léa, 60000 Beauvais (FR); VIN, Laetitia, 27140 Gisors (FR)
(74) Mandataire: Lavoix

(56) Documents cités:
- WO-A1-2020/028847
- US-B2- 9 561 358

## Description

La présente invention concerne une chambre implantable, propre à être implantée sous la peau d'un patient selon le préambule de la revendication 1.

La chambre implantable s'applique notamment à l'injection d'un liquide, tel qu'un médicament liquide ou un produit de diagnostic tel qu'un produit de contraste, dans le corps d'un patient, lors d'un traitement médical.

Certains traitements médicaux demandent des injections périodiques d'une dose médicamenteuse liquide dans le corps d'un patient. De telles injections périodiques sont connues par exemple pour l'oncologie, pour la dialyse, pour le traitement de l'hémophilie ou de maladies chroniques. Différents techniques d'imagerie médicale nécessitent aussi l'injection d'une substance de contraste.

Pour éviter de détériorer le système vasculaire du patient par des piquages répétitifs d'aiguille directement dans le système vasculaire du patient, il est connu d'insérer une chambre implantable du type précité sous la peau d'un patient, par exemple dans la poitrine, et de la raccorder au moyen d'un cathéter au système vasculaire du patient.

La chambre implantable est agencée sous la peau de telle sorte que le septum est orienté en regard de la peau, et la chambre implantable est maintenue en position.

Pour implanter une chambre du type précité, il est connu de réaliser une incision dans la peau du patient, puis d'insérer la chambre par sa pointe sous la peau du patient en poussant la chambre à travers l'incision.

L'extrémité en forme de pointe de la chambre facilite donc l'implantation, en permettant une entrée progressive de la chambre sous la peau. Néanmoins, dans certains cas, cette étape reste traumatique pour le patient, et peut conduire à une agression sur la peau du patient et/ou sur les tissus sous-cutanés.

WO 2020/028847 A1 divulgue une chambre implantable.

Un but de l'invention est d'obtenir une chambre implantable qui puisse facilement être insérée sous la peau d'un patient, avec un risque réduit de traumatisme pour le patient.

A cet effet, l'invention a pour objet une chambre implantable selon la revendication 1.

La chambre implantable selon l'invention peut comprendre l'une ou plusieurs des caractéristiques des revendications 2 à 10, prises isolément ou suivant toute combinaison techniquement possible.

L'invention a également pour objet un nécessaire de traitement selon la revendication 11.

L'invention sera mieux comprise à la lecture de la description qui va suivre, donnée uniquement à titre d'exemple, et faite en se référant aux dessins annexés, sur lesquels :
- [Fig 1] la figure 1 est une vue schématique d'un premier nécessaire de traitement comprenant une chambre implantable selon l'invention ;
- [Fig 2] la figure 2 est une vue prise en coupe suivant un plan vertical médian de la chambre implantable de la figure 1 ;
- [Fig 3] la figure 3 est une vue de dessous de la coque de la chambre implantable de la figure 2 ;
- [Fig 4] la figure 4 est une vue prise en coupe suivant un plan vertical médian de la coque de la chambre implantable de la figure 2 ;
- [Fig 5] la figure 5 est une vue analogue à la figure 4 d'une autre chambre implantable selon l'invention ;
- [Fig 6] la figure 6 est une vue analogue à la figure 4 d'encore une autre chambre implantable selon l'invention.

Dans tout ce qui suit, les termes « horizontal » et « vertical » s'entendent par rapport aux orientations de la chambre implantable lorsque sa surface inférieure est disposée sur une surface horizontale plane.

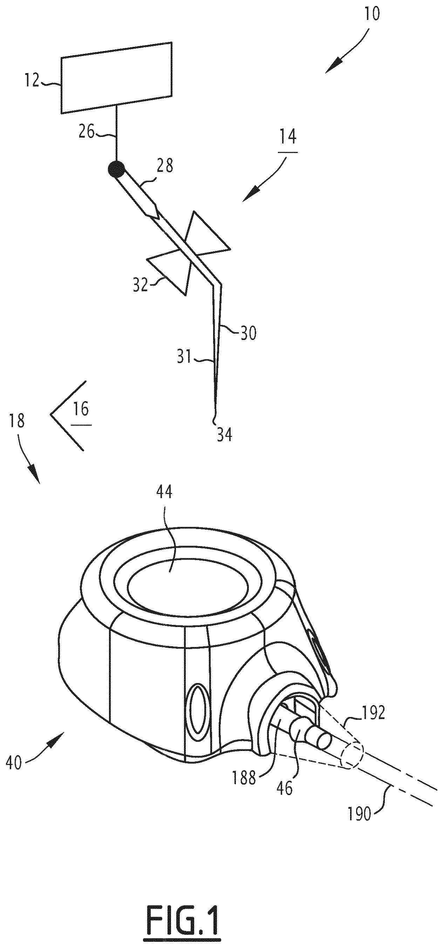

Un premier nécessaire 10 d'injection de liquide dans un patient est représenté sur la figure 1. Un tel nécessaire 10 est destiné notamment à injecter un liquide.

Le liquide injecté est par exemple un médicament. En variante, le liquide est un liquide non thérapeutique, tel qu'un produit de diagnostic, notamment un produit de contraste pour imagerie médicale. Le produit de contraste permet de réaliser une image du système vasculaire du patient, par exemple par tomodensitométrie (désignée par le terme anglais « computed tomography »).

Le nécessaire 10 comprend, d'amont en aval, un appareil d'injection 12 placé hors du corps du patient, un dispositif 14 d'injection à aiguille destiné à être inséré à travers la peau 16 du patient et une première chambre implantable 18 selon l'invention, disposée sous la peau 16 du patient et raccordée hydrauliquement en aval au système vasculaire du patient.

L'appareil d'injection 12 comprend par exemple une pompe à cylindre propre à distribuer un liquide avec un débit volumique réglable.

L'appareil d'injection 12 est raccordé au dispositif d'injection 14 par une tubulure de sortie 26.

Le dispositif à aiguille 14 peut comporter une tubulure souple 28 intermédiaire raccordée de manière démontable à la tubulure de sortie 26, une aiguille d'injection 30 propre à être piquée dans la peau du patient 16 pour pénétrer dans la chambre 18 et un mécanisme 32 de préhension de l'aiguille qui ne sera pas décrit en détail.

L'aiguille 30 est avantageusement une aiguille coudée dite de « Huber ». Elle comporte un corps tubulaire creux 31 définissant une lumière intérieure de circulation de produit. Elle est raccordée en amont à la tubulure intermédiaire 28. Elle présente en aval une extrémité libre 34 biseautée propre à faciliter l'introduction de l'aiguille 30 à travers la peau et dans la chambre 18.

L'aiguille 30 est avantageusement non carottante. Par « non carottante », on entend que l'aiguille 30 est apte à percer un septum sans produire de résidus solides se détachant du septum. Pour mesurer le caractère non carottant de l'aiguille 30, un test suivant l'annexe D de la Norme NF EN ISO 10555-6 d'Aout 2017 est par exemple mis en œuvre.

Le caractère non carottant de l'aiguille 30 est par exemple obtenu en disposant le biseau dans un plan parallèle à l'axe du corps tubulaire creux 31 au niveau de l'extrémité libre 34 de sorte que la lumière interne de l'aiguille ne soit pas visible lorsqu'on la regarde dans son axe principal.

En variante, le dispositif à aiguille 14 est du type décrit dans les demandes françaises FR 2 869 806 et FR 2 886 857 de la Demanderesse.

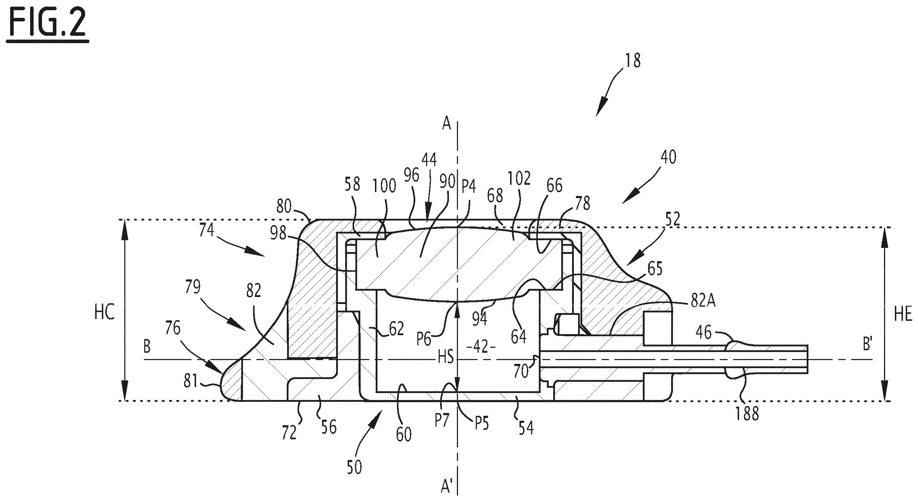

Comme illustré par les figures 1 à 4, la chambre 18 comporte un corps de base 40 délimitant un volume intérieur 42 de réception de liquide (voir figure 2), un septum 44 obturant le volume intérieur 42 et destiné à être piqué par l'aiguille 30, et un conduit 46 d'évacuation du liquide hors du volume intérieur 42 dans le corps du patient.

Le corps de base 40 comporte un récipient intérieur 50 d'un axe central A-A' et une coque périphérique extérieure 52 qui, dans cet exemple, est encliquetée sur le récipient intérieur 50.

Dans cet exemple, le récipient 50 comprend une cuve métallique 54 de réception de liquide, un insert inférieur 56 monté autour de la cuve 54 et une bague 58 de retenue du septum 44 disposée au-dessus de la cuve 54. En variante, la cuve 54, l'insert 56 et la bague 58 sont réalisés d'une seule pièce.

La cuve 54 est par exemple formée en titane. Elle comporte un fond 60 qui délimite le fond du volume intérieur et partiellement celui de la chambre 18 et une paroi latérale 62 sensiblement cylindrique qui délimite latéralement le volume intérieur 42.

La paroi latérale 62 présente un épaulement annulaire 64 d'appui du septum 44 qui s'étend le long de son bord supérieur. Cet épaulement définit une surface inférieure 65 de serrage du septum 44.

L'insert 56 s'étend autour de la paroi latérale 62 de la cuve, sous l'épaulement annulaire 64.

La bague 58 s'étend en regard de l'épaulement annulaire 64 et définit une surface supérieure 66 de serrage du septum 44.

Le volume intérieur 42 est délimité entre la paroi latérale 62 et le fond de la cuve 54. Le volume 42 s'ouvre vers le haut par une ouverture supérieure principale 68 recevant le septum 44. Il s'ouvre latéralement à travers la paroi latérale 62 de la cuve par une ouverture radiale 70 d'évacuation de produit. L'ouverture radiale 70 présente une section de passage inférieure à la section de passage de l'ouverture supérieure principale 68.

L'insert 56 délimite un passage radial à travers lequel s'étend le conduit 46.

La coque périphérique 52 est réalisée en matière plastique, par exemple en polyoxyméthylène ou POM. Elle s'étend autour de l'insert 56 et de la bague 58, pour maintenir en position la bague 58 et l'insert 56 sur la cuve 54.

La coque périphérique 52 présente une surface inférieure 72 qui affleure le fond 60 du récipient 50 et une surface latérale concave 74 qui s'étend de haut en bas depuis la bague 58 vers un bord périphérique inférieur 76 de la coque périphérique 52 situé au voisinage de la surface inférieure 72.

La coque périphérique 52 présente une lèvre supérieure périphérique 78 sensiblement parallèle à la surface inférieure 72.

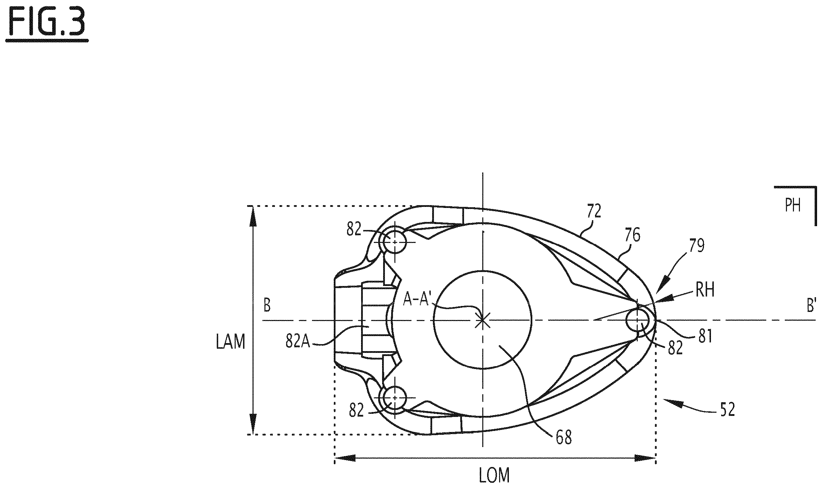

Le bord périphérique 76 délimite le contour extérieur de la chambre 18. Dans cet exemple, comme illustré par la figure 3, ce contour est de forme sensiblement triangulaire.

Ainsi, la coque périphérique 52 présente, à l'opposé du conduit d'évacuation 46, une pointe 79 qui fait saillie par rapport à la lèvre périphérique 78 suivant l'axe B-B' du conduit d'évacuation 46 jusqu'à une extrémité libre 81.

La lèvre périphérique 78 fait saillie radialement vers l'axe A-A' autour de l'ouverture supérieure principale 68. Elle s'appuie ici sur la bague 58, autour de l'ouverture supérieure principale 68.

La lèvre périphérique 78 définit, avec la surface latérale 74 un bord supérieur arrondi 80 qui s'étend sur toute la périphérie de la lèvre 78.

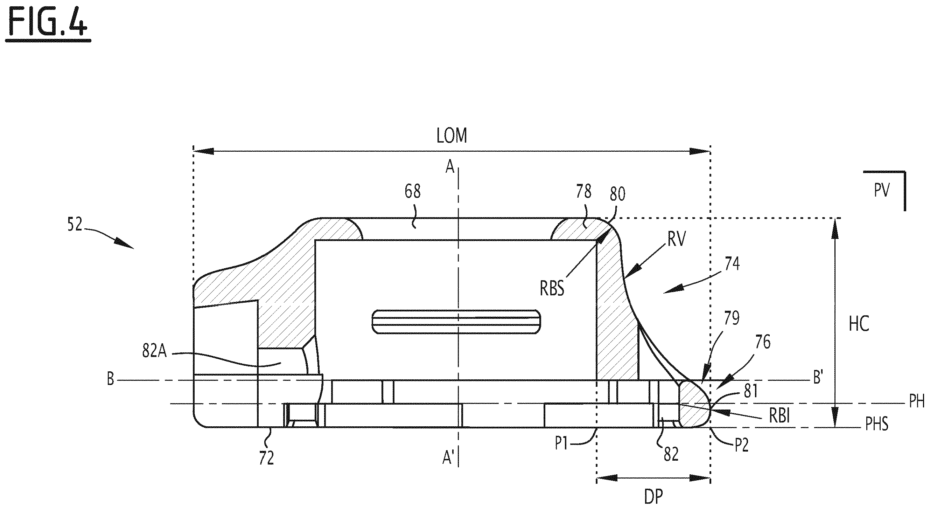

Avantageusement, en référence à la figure 4, le rapport R1 entre le rayon de courbure RBS du bord supérieur arrondi 80 et la hauteur maximale HC de la coque périphérique 52, prise verticalement entre la surface inférieure 72 et la lèvre périphérique 78, au niveau de la projection orthogonale P1 de la lèvre annulaire 78 sur la surface inférieure 72, la plus proche de l'extrémité libre 81, est compris entre 0,15 et 0,21. Dans l'exemple représenté sur la figure 4, le rapport R1 est compris entre 0,15 et 0,20, notamment entre 0,15 et 0,17.

La projection orthogonale P1 est ici prise au niveau du point à partir duquel la lèvre périphérique 78 commence à faire saillie vers l'axe A-A'.

Le rayon de courbure RBS du bord supérieur arrondi 80 est de préférence compris entre 1,5 mm et 2,5 mm, notamment entre 1,7 mm et 2,1 mm. Dans l'exemple représenté sur la figure 4, le rayon de courbure RBS est compris entre 1,9 mm et 2,1 mm.

Le bord supérieur arrondi 80 présentant les dimensions précitées limite l'agressivité pour la peau du patient, lors de l'insertion de la chambre 18 sous la peau du patient et pendant la durée d'implantation.

Le bord périphérique inférieur 76 est convexe. Il est arrondi sur toute la périphérie de la coque périphérique 52.

Avantageusement, le rapport R2 entre le rayon de courbure RBI du bord périphérique inférieur 76, et la hauteur maximale HC de la coque périphérique 52, telle que définie plus haut, est compris entre 0,06 et 0,10. Dans l'exemple représenté sur la figure 4, le rapport R2 est compris entre 0,06 et 0,08, notamment entre 0,06 et 0,07.

Le rayon de courbure RBI du bord périphérique inférieur 76 est de préférence compris entre 0,5 mm et 1,1 mm, notamment entre 0,7 mm et 0,9 mm.

De même, le bord périphérique inférieur 76 présentant les dimensions précitées limite l'agressivité pour la peau du patient et pour les tissus sous-cutanés, lors de l'insertion de la chambre 18 sous la peau du patient.

La pointe 79 est incurvée verticalement et horizontalement.

En référence à la figure 4, dans un plan vertical médian PV contenant l'extrémité libre 81, le plan PV passant ici par l'axe B-B' et par l'axe A-A', la pointe 79 présente un rayon de courbure vertical RV pris sur la surface latérale 74 entre le bord supérieur 80 et le bord inférieur 76.

Selon l'invention, le rapport R3 entre le rayon de courbure vertical RV de la pointe 79 et la distance DP séparant la projection P1 définie plus haut et la projection P2 de l'extrémité libre 81 sur un plan horizontal PHS défini par la surface inférieure 72, est compris entre 2,0 et 4,0. Avantageusement, le rapport R3 est compris entre 2,3 et 2,7, et dans l'exemple de la figure 4, entre 2,5 et 2,6.

Ceci garantit une insertion très efficace de la pointe 79 sous la peau, tout en limitant la taille de l'incision nécessaire, et l'agressivité sur la peau.

Avantageusement, le rayon de courbure vertical RV est compris entre 8 mm et 25 mm, notamment entre 9 mm et 15 mm. Dans l'exemple de la figure 4, le rayon de courbure vertical RV est compris entre 13 mm et 15 mm.

Dans un plan horizontal PH passant par l'extrémité libre 81, parallèlement à la surface inférieure 72, la pointe 79 présente un rayon de courbure horizontal RH, comme visible sur la figure 3.

Le rapport R4 entre le rayon de courbure vertical RV et le rayon de courbure horizontal RH est compris entre 0,3 et 1,5, notamment entre 0,3 et 0,6. Dans l'exemple de la figure 4, le rapport R4 est compris entre 0,5 et 0,6. Ceci limite également l'agressivité sur la peau.

Avantageusement, le rayon de courbure horizontal RH est compris entre 15 mm et 30 mm, de préférence entre 19 mm et 26 mm.

Dans l'exemple représenté sur la figure 4, le rayon de courbure horizontal RH est compris entre 24 mm et 26 mm.

Plus généralement, le rapport R5 entre la hauteur maximale HC de la coque périphérique 52, tel que définie plus haut, et la largeur maximale LAM de la coque périphérique 52, prise au niveau du bord périphérique inférieur 76, perpendiculairement à l'axe B-B' est compris entre 0,45 et 0,55. Dans l'exemple de la figure 4, ce rapport R5 est compris entre 0,53 et 0,55.

De même, le rapport R6 de la longueur maximale LOM de la coque périphérique 52, prise le long de l'axe B-B', à la largeur maximale LAM, tel que définie plus haut est compris entre 1,20 et 1,40. Dans l'exemple de la figure 4, le rapport R6 est compris entre 1,35 et 1,38.

Ces rapports R5 et R6 diminuent encore la taille de l'incision, et augmentent la stabilité de la chambre implantable 18 sous la peau.

La coque périphérique 52 délimite en outre des ouvertures traversantes 82 de passage d'un fil de suture et une ouverture radiale 82A de passage du conduit d'évacuation de liquide 46.

Les ouvertures de passage 82 raccordent la surface latérale 74 à la surface inférieure 72 au voisinage du bord périphérique 76, sensiblement parallèlement à l'axe A-A'. Elles sont réparties angulairement autour de l'axe A-A'.

Le diamètre maximal des ouvertures de passage 82 est avantageusement compris entre 1,5 mm et 2,5 mm, notamment entre 1,6 mm et 2,1 mm. Ceci prévient l'adhésion cellulaire au sein des ouvertures, et facilite le retrait de la chambre implantable 18 lorsqu'il est nécessaire de la retirer.

L'ouverture radiale 82A débouche en regard de l'ouverture d'évacuation 70 qu'elle prolonge radialement par rapport à l'axe A-A'.

En référence à la figure 2, le septum 44 comporte un bloc 90 de base, réalisé en un matériau étanche. Le septum 44 comporte avantageusement une composition antimicrobienne destinée à s'appliquer sur l'aiguille 30 lors du piquage de l'aiguille à travers le bloc 90, par exemple un revêtement antimicrobien couvrant au moins une surface extérieure du bloc 90.

Le bloc 90 est réalisé avantageusement à partir d'une matrice faite en un matériau étanche continu et plein. Ce matériau est par exemple une matière plastique souple, telle que du silicone. Le bloc 90 présente un contour extérieur sensiblement homothétique de celui de l'ouverture supérieure principale 68.

Le bloc 90 s'étend sensiblement transversalement par rapport à un axe central A-A' d'insertion de l'aiguille dans le septum 44. Il présente une hauteur maximale, prise parallèlement à l'axe A-A', inférieure à son étendue transversale maximale, prise perpendiculairement à l'axe A-A'.

Dans l'exemple représenté sur la figure 1, le septum 44 est formé par un disque d'axe A-A' présentant une surface transversale inférieure 94, destinée à obturer vers le haut le volume intérieur 42, une surface transversale supérieure 96 opposée à la surface inférieure 94 et destinée à être placée en regard de la peau du patient, et une surface périphérique 98 raccordant entre elles les surfaces 94 et 96.

Lors du montage du septum 44 dans la chambre 18, le bloc 90 est propre à passer d'une configuration de repos à une configuration partiellement comprimée lorsqu'il est monté dans le corps 40.

Dans la configuration comprimée, le septum 44 est inséré à sa périphérie entre la surface transversale inférieure 65 définie par l'épaulement d'appui 64 et la surface transversale supérieure 66 définie par la bague de retenue 58.

Le bloc 90 comporte ainsi une région périphérique 100 comprimée et une région centrale 102 relativement moins comprimée.

Dans l'exemple représenté sur la figure 2, la région périphérique comprimée 100 présente une compression axiale, prise sur la hauteur du septum 44 entre les surfaces 95 et 96 par rapport à la configuration de repos, supérieure à 20 %, et avantageusement inférieure à 50 %. Cette compression est par exemple comprise entre 30 % et 50 %, et avantageusement comprise entre 34 % et 43 %.

Ainsi, dans la configuration comprimée, la hauteur moyenne du septum 44 dans la région comprimée 100 est inférieure à 80% et est notamment supérieure à 50% de la hauteur moyenne du septum 44 dans la même région 100 dans la configuration de repos, ou de la hauteur moyenne du septum 44 prise au niveau de l'axe A-A' dans la région centrale moins comprimée 102.

En référence à la figure 2, le rapport R7 entre la hauteur émergente HE du septum 44, définie par la hauteur séparant le point P4 du septum 44 le plus éloigné de la surface inférieure 72 et la projection orthogonale P5 du point P4 sur la surface inférieure 72, et la hauteur maximale HC de la coque périphérique 52, telle que définie précédemment, est compris entre 0,95 et 1,05 , notamment entre 0,98 et 1,02 . Dans l'exemple de la figure 2, le rapport R7 est compris entre 0,98 et 1,00.

Ce rapport évite que le septum 44 ne soit trop saillant, ce qui facilite la palpation de la lèvre périphérique 78 à travers la peau pour positionner plus facilement l'aiguille 30, notamment pour des patients minces.

La hauteur émergente HE est par exemple comprise entre 5 mm et 15 mm, notamment entre 8 mm et 13 mm.

La hauteur sous septum HS séparant le point P6 du septum 44 le plus proche du fond 60 de la cuve de sa projection orthogonale P7 sur le fond 60 de la cuve est comprise entre 2 mm et 6 mm, notamment entre 3 mm et 5 mm.

Ceci permet d'accueillir entièrement l'extrémité libre 34 de l'aiguille 30 dans le volume intérieur 42, pour assurer un débit d'injection adéquat.

La région périphérique comprimée 100 s'étend sur une couronne de largeur représentant moins de 35 % et avantageusement plus de 15 % de l'étendue transversale maximale du septum 44, prise perpendiculairement à l'axe A-A'.

De préférence, le rapport R8 entre le diamètre maximal du septum 44, pris perpendiculairement à l'axe A-A' et le diamètre découvert du septum 44, situé à l'intérieur de la lèvre périphérique 78 est compris entre 1,20 et 1,40, notamment entre 1,22 et 1,37. Dans l'exemple de la figure 2, ce rapport R8 est compris entre 1,30 et 1,37.

Par ailleurs, le rapport R9 entre le diamètre découvert du septum 44, et la largeur maximale LAM de la coque périphérique 52, tel que définie plus haut, est compris entre 0,40 et 0,60, notamment entre 0,45 et 0,55. Dans l'exemple de la figure 2, le rapport R9 est compris entre 0,51 et 0,55.

La surface occupée par la région périphérique comprimée 100, prise en projection dans un plan perpendiculaire à l'axe A-A' est ainsi inférieure à 50 %, et est par exemple comprise entre 30 % et 50 % de la surface totale occupée par le septum, prise en projection perpendiculairement à l'axe A-A'.

Ainsi, plus de 10% et avantageusement moins de 20 %, notamment entre 12 % et 15 % du volume du septum 44 dans la configuration de repos est déplacé vers un plan transversal central du septum 44 dans la configuration comprimée.

En référence à la figure 1, le conduit d'évacuation 46 comprend un raccord rigide 188, une tubulure souple 190 et une bague de sertissage 192 de la tubulure souple 190 sur le raccord rigide 188.

Il fait saillie radialement par rapport au corps de base 40 à travers l'ouverture radiale 82A et au-delà de cette ouverture radiale 82A.

Le raccord rigide 188 est formé par une tige creuse assemblée sur la paroi extérieure 62 du récipient autour de l'ouverture d'évacuation 70. La tige creuse fait saillie radialement à travers l'ouverture radiale 82A hors du corps de base 40.

La tubulure 190 est réalisée à base d'une matière plastique souple. Elle présente une longueur supérieure à l'étendue transversale maximale du corps de base 40, par exemple au moins deux fois supérieure à cette étendue maximale.

La tubulure 190 s'étend entre une extrémité amont engagée en force autour de l'extrémité libre du raccord rigide 188 et une extrémité libre aval destinée à être placée dans le conduit de circulation du sang.

La bague de sertissage 192 est montée en force dans l'ouverture radiale 82A pour sertir la tubulure souple 190 contre la tige creuse 188. Elle fait saillie radialement au-delà du corps de base 40 sur une longueur inférieure à celle de la tubulure 190, par exemple au moins deux fois inférieure à celle de la tubulure 190.

Pour utiliser le nécessaire 10, la chambre implantable 18 est tout d'abord disposée sous la peau du patient. À cet effet, une incision est réalisée dans la peau du patient, et la chambre 18 est introduite sous la peau à travers l'incision, par sa pointe 79.

La pointe 79 présentant un rapport R3 du rayon de courbure vertical RV de la pointe 79, à la distance DP compris entre 2,0 et 4,0 et avantageusement, un rapport R4 du rayon de courbure vertical RV au rayon de courbure horizontal RH compris entre 0,3 et 1,5, son insertion dans l'incision est favorisée, même si l'incision est de petite taille, tout en limitant au minimum les dommages sur la peau.

Ceci est particulièrement le cas lorsque le rapport R1 du rayon de courbure RBS du bord supérieur arrondi 80 à la hauteur maximale HC de la coque périphérique 52 est compris entre 0,15 et 0,21 et avantageusement, lorsque le rapport R2 du rayon de courbure RBI du bord périphérique inférieur 76 à la hauteur maximale HC de la coque périphérique 52 est compris entre 0,06 et 0,10.

Ainsi, l'implantation de la chambre implantable 18 est très peu invasive et reste simple à réaliser.

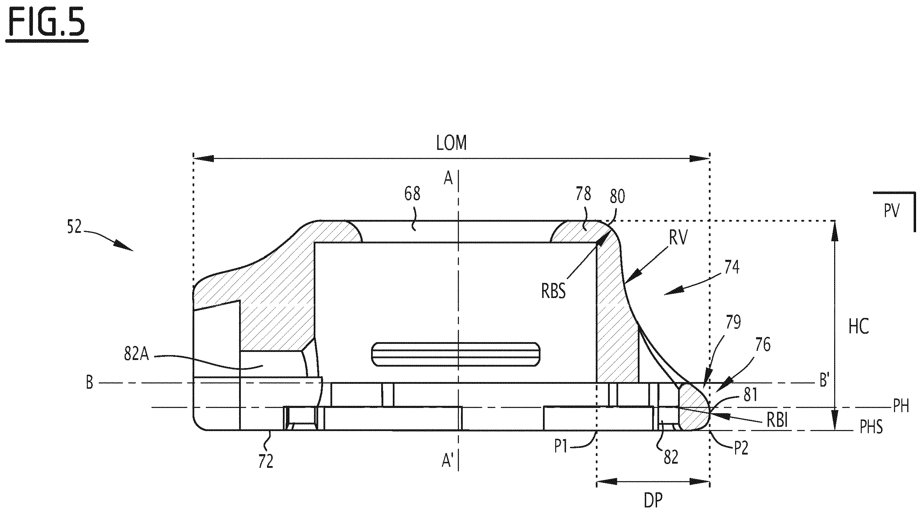

La chambre implantable 18 représentée sur la figure 5 diffère de celle illustrée sur la figure 4 en ce que le rapport R1 du rayon de courbure RBS du bord supérieur arrondi 80 à la hauteur maximale HC de la coque périphérique 52 est compris entre 0,19 et 0,20.

Le rapport R2 du rayon de courbure RBI du bord périphérique inférieur 76 à la hauteur maximale HC de la coque périphérique 52, est compris entre 0,07 et 0,08.

Le rapport R3 du rayon de courbure vertical RV de la pointe 79 à la distance DP est compris entre 2,4 et 2,5.

Le rapport R4 du rayon de courbure vertical RV au rayon de courbure horizontal RH est compris 0,35 et 0,45.

Le rapport R5 de la hauteur maximale HC de la coque périphérique 52 à la largeur maximale LAM de la coque périphérique 52 est compris entre 0,47 et 0,51.

De même, le rapport R6 de la longueur maximale LOM de la coque périphérique 52 à la largeur maximale LAM de la coque périphérique 52 est compris entre 1,20 et 1,25.

Le rapport R7 entre la hauteur émergente HE du septum 44 et la hauteur maximale HC de la coque périphérique 52 est compris entre 0,97 et 0,99.

Le rapport R8 entre le diamètre maximal du septum 44 et le diamètre découvert du septum 44 est compris entre 1,20 et 1,30.

Le rapport R9 entre le diamètre découvert du septum 44, et la largeur maximale LAM de la coque périphérique 52 est compris entre 0,48 et 0,51.

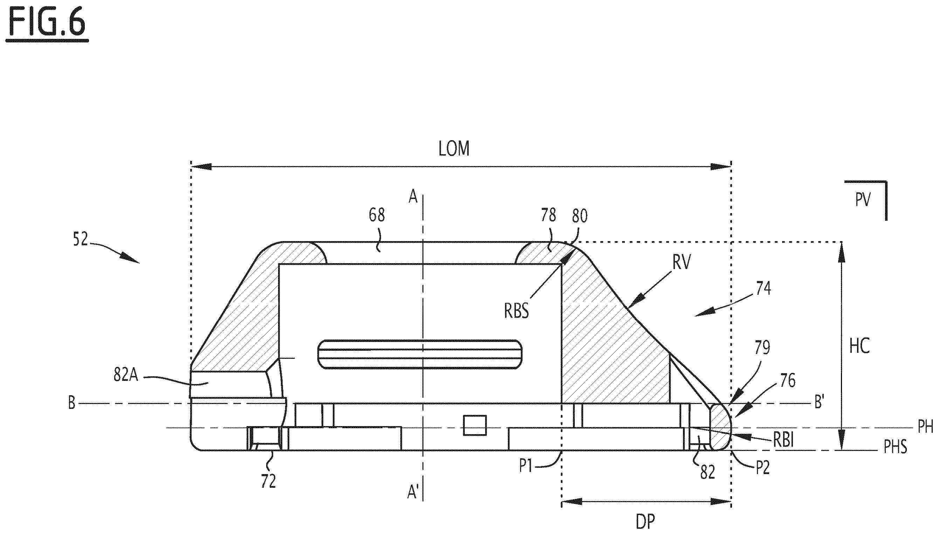

La chambre implantable 18 représentée sur la figure 6 diffère de celle illustrée sur la figure 4 en ce que le rapport R1 du rayon de courbure RBS du bord supérieur arrondi 80 à la hauteur maximale HC de la coque périphérique 52, est compris entre 0,20 et 0,21.

Le rapport R2 du rayon de courbure RBI du bord périphérique inférieur 76 à la hauteur maximale HC de la coque périphérique 52, est compris entre 0,08 et 0,10.

Le rapport R3 du rayon de courbure vertical RV de la pointe 79 à la distance DP est compris entre 3,5 et 3,7.

Le rapport R4 du rayon de courbure vertical RV au rayon de courbure horizontal RH est compris 1,0 et 1,2.

Le rapport R5 de la hauteur maximale HC de la coque périphérique 52 à la largeur maximale LAM de la coque périphérique 52 est compris entre 0,49 et 0,52.

De même, le rapport R6 de la longueur maximale LOM de la coque périphérique 52 à la largeur maximale LAM est compris entre 1,25 et 1,35.

Le rapport R7 entre la hauteur émergente HE du septum 44 et la hauteur maximale HC de la coque périphérique 52 est compris entre 1,01 et 1,03.

Le rapport R8 entre le diamètre maximal du septum 44 et le diamètre découvert du septum 44 est compris entre 1,20 et 1,30.

Le rapport R9 entre le diamètre découvert du septum 44, et la largeur maximale LAM de la coque périphérique 52 est compris entre 0,44 et 0,48.

## Revendications

1. Chambre implantable (18), propre à être implantée sous la peau d'un patient, comprenant :
- un corps de base (40) creux délimitant un volume intérieur (42) de circulation du liquide, le volume intérieur (42) débouchant par une ouverture supérieure principale (68) ;
- un septum (44) obturant l'ouverture supérieure principale (68), le septum (44) étant destiné à être piqué par une aiguille (30) ;
- un conduit d'évacuation (46) de liquide hors du volume intérieur (42) faisant saillie à l'écart du corps de base (40), le corps de base (40) comportant un récipient intérieur (50) et une coque périphérique (52) extérieure assemblée sur le récipient intérieur (50), le conduit d'évacuation (46) s'étendant à travers un passage radial du récipient intérieur (50), la coque périphérique (52) présentant, autour de l'ouverture supérieure principale (68), une lèvre périphérique (78), et à l'opposé du conduit d'évacuation (46), une pointe (79) s'étendant entre la lèvre périphérique (78) et une surface inférieure (72) de la coque périphérique (52), en saillie par rapport à la lèvre périphérique (78), jusqu'à une extrémité libre (81) ;
**caractérisée en ce que**, dans un plan vertical médian contenant l'extrémité libre (81), le rapport (R3) entre :
- le rayon de courbure vertical (RV) de la pointe (79), pris sur une surface latérale concave (74) en s'étendant entre la lèvre périphérique (78) et l'extrémité libre (81) ; et
- la distance (DP) séparant la projection (P1) de la lèvre périphérique (78) sur la surface inférieure (72) la plus proche de l'extrémité libre (81) et la projection (P2) de l'extrémité libre (81) sur un plan défini par la surface inférieure (72) est compris entre 2,0 et 4,0.

2. Chambre implantable (18) selon la revendication 1, dans laquelle le rapport (R3) entre :
- le rayon de courbure vertical (RV) de la pointe (79), pris entre la lèvre périphérique (78) et l'extrémité libre (81) ; et
- la distance (DP) séparant la projection (P1) de la lèvre périphérique (78) sur la surface inférieure (72) la plus proche de l'extrémité libre (81) et la projection (P2) de l'extrémité libre (81) sur un plan défini par la surface inférieure (72) est compris entre 2,3 et 2,7.

3. Chambre implantable (18) selon la revendication 1, dans laquelle le rapport (R3) entre :
- le rayon de courbure vertical (RV) de la pointe (79), pris entre la lèvre périphérique (78) et l'extrémité libre (81) ; et
- la distance (DP) séparant la projection (P1) de la lèvre périphérique (78) sur la surface inférieure (72) la plus proche de l'extrémité libre (81) et la projection (P2) de l'extrémité libre (81) sur un plan défini par la surface inférieure (72) est compris entre 3,5 et 3,7.

4. Chambre implantable (18) selon l'une quelconque des revendications précédentes, dans laquelle la coque périphérique (52) définit, le long de la surface inférieure (72) au moins autour de la pointe (79), un bord périphérique inférieur (76) arrondi, le rapport (R2) entre le rayon de courbure (RBI) du bord périphérique inférieur (76), et la hauteur maximale (HC) de la coque périphérique (52) étant compris entre 0,06 et 0,10.

5. Chambre implantable (18) selon l'une quelconque des revendications précédentes, dans laquelle la coque périphérique (52) extérieure définit, le long de la lèvre périphérique (78) au moins en regard de la pointe (79), un bord supérieur arrondi (80), le rapport (R1) entre le rayon de courbure (RBS) du bord supérieur arrondi (80), et la hauteur maximale (HC) de la coque périphérique (52) étant compris entre 0,15 et 0,21.

6. Chambre (18) selon l'une quelconque des revendications précédentes, dans laquelle la pointe (79) présente un rayon de courbure horizontal (RH) au niveau de l'extrémité libre (81), le rapport (R4) du rayon de courbure vertical (RV) au rayon de courbure horizontal (RH) étant compris entre 0,3 et 1,5

7. Chambre implantable (18) selon l'une quelconque des revendications précédentes, dans laquelle le rapport (R5) entre la hauteur maximale (HC) de la coque périphérique (52) et la largeur maximale (LAM) de la coque périphérique (52) est compris entre 0,45 et 0,55.

8. Chambre implantable (18) selon l'une quelconque des revendications précédentes, dans laquelle le rapport (R6) entre la longueur maximale (LOM) de la coque périphérique (52) et la largeur maximale (LAM) de la coque périphérique (52) est compris entre 1,20 et 1,40.

9. Chambre implantable (18) selon l'une quelconque des revendications précédentes, dans laquelle le rapport (R7) entre la hauteur du point (P4) le plus haut du septum (44), prise depuis la surface inférieure (72) et la hauteur maximale (HC) de la coque périphérique (52) est compris entre 0,95 et 1,05.

10. Chambre implantable (18) selon l'une quelconque des revendications précédentes, dans laquelle la hauteur séparant le point (P6) du septum le plus proche du fond (60) du volume intérieur (42) et le fond du volume intérieur (42) est supérieure à 2 mm.

11. Nécessaire de traitement (10) comportant :
- une chambre implantable (18) selon l'une quelconque des revendications précédentes ;
- une aiguille (30), en particulier une aiguille de Huber destinée à être plantée dans le septum (44) de la chambre implantable (18).

## Patentansprüche

1. Implantierbare Kammer (18), die geeignet ist, um unter die Haut eines Patienten implantiert zu werden, umfassend:
- einen hohlen Basiskörper (40), der ein inneres Volumen (42) für die Zirkulation der Flüssigkeit begrenzt, wobei das innere Volumen (42) durch eine obere Hauptöffnung (68) mündet;
- ein Septum (44), das die obere Hauptöffnung (68) verschließt, wobei das Septum (44) ausgelegt ist, um von einer Nadel (30) eingestochen zu werden;
- eine Auslassleitung (46) von Flüssigkeit aus dem inneren Volumen (42), die aus dem Basiskörper (40) vorspringt, wobei der Basiskörper (40) einen inneren Behälter (50) und eine äußere Schale (52) umfasst, die auf dem inneren Behälter (50) zusammengebaut ist, wobei sich die Auslassleitung (46) durch einen radialen Durchgang des inneren Behälters (50) erstreckt, wobei die Umfangsschale (52) um die obere Hauptöffnung (68) eine Umfangslippe (78) und gegenüber der Auslassleitung (46) eine Spitze (79) aufweist, die sich zwischen der Umfangslippe (78) und einer unteren Fläche (72) der Umgangsschale (52) mit Bezug auf die Umfangslippe (78) bis zu einem freien Ende (81) vorstehend erstreckt;
**dadurch gekennzeichnet, dass** auf einer mittleren vertikalen Ebene, die das freie Ende (81) enthält, das Verhältnis (R3) zwischen:
- dem vertikalen Krümmungsradius (RV) der Spitze (79), gemessen auf einer konkaven Seitenfläche (74), die sich zwischen der Umfangslippe (78) und dem freien Ende (81) erstreckt; und
- dem Abstand (DP), der die Projektion (P1) der Umfangslippe (78) auf der unteren Fläche (72), die dem freien Ende (81) am nächsten liegt, und der Projektion (P2) des freien Endes (81) auf einer Ebene, die durch die untere Fläche (72) definiert ist, im Bereich zwischen 2,0 und 4,0 liegt.

2. Implantierbare Kammer (18) nach Anspruch 1, wobei das Verhältnis (R3) zwischen:
- dem vertikalen Krümmungsradius (RV) der Spitze (79), gemessen zwischen der Umfangslippe (78) und dem freien Ende (81); und
- dem Abstand (DP), der die Projektion (P1) der Umfangslippe (78) auf der unteren Fläche (72), die dem freien Ende (81) am nächsten liegt, und der Projektion (P2) des freien Endes (81) auf einer Ebene, die durch die untere Fläche (72) definiert ist, im Bereich zwischen 2,3 und 2,7 liegt.

3. Implantierbare Kammer (18) nach Anspruch 1, wobei das Verhältnis (R3) zwischen:
- dem vertikalen Krümmungsradius (RV) der Spitze (79), gemessen zwischen der Umfangslippe (78) und dem freien Ende (81); und
- dem Abstand (DP), der die Projektion (P1) der Umfangslippe (78) auf der unteren Fläche (72), die dem freien Ende (81) am nächsten liegt, und der Projektion (P2) des freien Endes (81) auf einer Ebene, die durch die untere Fläche (72) definiert ist, im Bereich zwischen 3,5 und 3,7 liegt.

4. Implantierbare Kammer (18) nach einem der vorhergehenden Ansprüche, wobei die Umfangsschale (52) entlang der unteren Fläche (72), mindestens um die Spitze (79), einen abgerundeten unteren Umfangsrand (76) definiert, wobei das Verhältnis (R2) zwischen dem Krümmungsradius (RBI) des unteren Umfangsrands (76) und der maximalen Höhe (HC) der Umfangsschale (52) im Bereich zwischen 0,06 und 0,10 liegt.

5. Implantierbare Kammer (18) nach einem der vorhergehenden Ansprüche, wobei die äußere Umfangsschale (52) entlang der Umfangslippe (78), mindestens gegenüber der Spitze (79), einen abgerundeten oberen Rand (80) definiert, wobei das Verhältnis (R1) zwischen dem Krümmungsradius (RBS) des abgerundeten oberen Rands (80) und der maximalen Höhe (HC) der Umfangsschale (52) im Bereich zwischen 0,15 und 0,21 liegt.

6. Kammer (18) nach einem der vorhergehenden Ansprüche, wobei die Spitze (79) auf der Ebene des freien Endes (81) einen horizontalen Krümmungsradius (RH) aufweist, wobei das Verhältnis (R4) des vertikalen Krümmungsradius (RV) zum horizontalen Krümmungsradius (RH) im Bereich zwischen 0,3 und 1,5 liegt.

7. Implantierbare Kammer (18) nach einem der vorhergehenden Ansprüche, wobei das Verhältnis (R5) zwischen der maximalen Höhe (HC) der Umfangsschale (52) und der maximalen Breite (LAM) der Umfangsschale (52) im Bereich zwischen 0,45 und 0,55 liegt.

8. Implantierbare Kammer (18) nach einem der vorhergehenden Ansprüche, wobei das Verhältnis (R6) zwischen der maximalen Länge (LOM) der Umgangsschale (52) und der maximalen Breite (LAM) der Umfangsschale (52) im Bereich zwischen 1,20 und 1,40 liegt.

9. Implantierbare Kammer (18) nach einem der vorhergehenden Ansprüche, wobei das Verhältnis (R7) zwischen der Höhe des höchsten Punktes (P4) des Septums (44), gemessen von der unteren Fläche (72), und der maximalen Höhe (HC) der Umfangsschale (52) im Bereich zwischen 0,95 und 1,05 liegt.

10. Implantierbare Kammer (18) nach einem der vorhergehenden Ansprüche, wobei die Höhe, die den Punkt (P6) des Septums, der dem Boden (60) des inneren Volumens (42) am nächsten liegt, und den Boden des inneren Volumens (42) trennt, mehr als 2 mm beträgt.

11. Behandlungsnecessaire (10), umfassend:
- eine implantierbare Kammer (18) nach einem der vorhergehenden Ansprüche;
- eine Nadel (30), insbesondere eine Huber-Nadel, die ausgelegt ist, um in das Septum (44) der implantierbaren Kammer (18) eingesetzt zu werden.

## Claims

1. An implantable chamber (18), able to be implanted under the skin of a patient, comprising:
- a hollow base body (40) delimiting an interior volume (42) for fluid circulation, the interior volume (42) opening through a main upper opening (68);
- a septum (44) sealing the main upper opening (68), the septum (44) being intended to be pierced by a needle (30);
- a fluid outlet conduit (46) from the interior volume (42) protruding away from the base body (40), the base body (40) including an inner container (50) and an outer peripheral shell (52) assembled on the inner container (50), the outlet conduit (46) extending through a radial passage of the inner container (50), the peripheral shell (52) presenting, around the main upper opening (68), a peripheral lip (78), and opposite the outlet conduit (46), a tip (79) extending between the peripheral lip (78) and a lower surface (72) of the peripheral shell (52), protruding relative to the peripheral lip (78), to a free end (81)
**characterized in that**, in a vertical median plane containing the free end (81), the ratio (R3) between:
- the vertical radius of curvature (RV) of the tip (79), taken on a concave lateral surface (74) extending between the peripheral lip (78) and the free end (81); and
- the distance (DP) separating the projection (P1) of the peripheral lip (78) on the lower surface (72) closest to the free end (81) and the projection (P2) of the free end (81) on a plane defined by the lower surface (72) is between 2.0 and 4.0.

2. The implantable chamber (18) according to claim 1, wherein the ratio (R3) between:
- the vertical radius of curvature (RV) of the tip (79), taken between the peripheral lip (78) and the free end (81); and
- the distance (DP) separating the projection (P1) of the peripheral lip (78) on the lower surface (72) closest to the free end (81) and the projection (P2) of the free end (81) on a plane defined by the lower surface (72) is between 2.3 and 2.7.

3. The implantable chamber (18) according to claim 1, wherein the ratio (R3) between:
- the vertical radius of curvature (RV) of the tip (79), taken between the peripheral lip (78) and the free end (81); and
- the distance (DP) separating the projection (P1) of the peripheral lip (78) on the lower surface (72) closest to the free end (81) and the projection (P2) of the free end (81) on a plane defined by the lower surface (72) is between 3.5 and 3.7.

4. The implantable chamber (18) according to any one of the preceding claims, wherein the peripheral shell (52) defines, along the lower surface (72) at least around the tip (79), a rounded lower peripheral edge (76), the ratio (R2) between the radius of curvature (RBI) of the lower peripheral edge (76), and the maximum height (HC) of the peripheral shell (52) being between 0.06 and 0.10.

5. The implantable chamber (18) according to any one of the preceding claims, wherein the outer peripheral shell (52) defines, along the peripheral lip (78) at least facing the tip (79), a rounded upper edge (80), the ratio (R1) between the radius of curvature (RBS) of the rounded upper edge (80), and the maximum height (HC) of the peripheral shell (52) being between 0.15 and 0.21.

6. The chamber (18) according to any one of the preceding claims, wherein the tip (79) presents a horizontal radius of curvature (RH) at the free end (81), the ratio (R4) of the vertical radius of curvature (RV) to the horizontal radius of curvature (RH) being between 0.3 and 1.5.

7. The implantable chamber (18) according to any one of the preceding claims, wherein the ratio (R5) between the maximum height (HC) of the peripheral shell (52) and the maximum width (LAM) of the peripheral shell (52) is between 0.45 and 0.55.

8. The implantable chamber (18) according to any one of the preceding claims, wherein the ratio (R6) between the maximum length (LOM) of the peripheral shell (52) and the maximum width (LAM) of the peripheral shell (52) is between 1.20 and 1.40.

9. The implantable chamber (18) according to any one of the preceding claims, wherein the ratio (R7) between the height of the highest point (P4) of the septum (44), taken from the lower surface (72) and the maximum height (HC) of the peripheral shell (52) is between 0.95 and 1.05.

10. The implantable chamber (18) according to any one of the preceding claims, wherein the height separating the point (P6) of the septum the closest to the bottom (60) of the interior volume (42) and the bottom of the interior volume (42) is greater than 2 mm.

11. A treatment kit (10) including:
- an implantable chamber (18) according to any one of the preceding claims;
- a needle (30), particularly a Huber needle intended to be inserted into the septum (44) of the implantable chamber (18).
